# EUROPEAN PATENT SPECIFICATION

(11) **EP 1 874 336 B1**
(45) Date of publication and mention of the grant of the patent: **27.04.2016**
(21) Application number: 06740067.1
(22) Date of filing: 28.03.2006
(51) Int. Cl.: A61K 38/17, A23L 29/00, A61K 31/70

(54) **A METHOD OF IMPROVING LEARNING AND MEMORY IN AN INFANT COMPRISING ADMINISTERING CASEIN GLYCOMACROPEPTIDE**
VERFAHREN ZUR VERBESSERUNG DER LERN- UND GEDÄCHNTISFUNKTION VON KLEINKINDERN MITTELS VERABREICHUNG VON CASEIN GLYCOMACROPEPTIDE
PROCEDE D'AMELIORATION DE L'APPRENTISSAGE ET DE LA MEMOIRE CHEZ LES MAMMIFERES COMPRENANT L'ADMINISTRATION DE CASEIN GLYCOMACROPEPTIDE

(30) Priority: 29.04.2005 US 118790
(43) Date of publication of application: 09.01.2008
(73) Proprietor: MJN U.S. Holdings LLC, Glenview, IL 60026 (US); THE UNIVERSITY OF SYDNEY, Sydney, NSW 2006 (AU)
(72) Inventor: MCMAHON, Robert J., Evansville, Indiana 47721-0001 (US); WANG, Bing, Wolli Creek, New South Wales 2205 (AU); RUMSEY, Steven Charles, Curitiba, PA 80250-080 (BR); BRAND-MILLER, Jennie, Greenwich, New South Wales 2065 (AU)
(74) Representative: V.O.
(86) International application number: PCT/US2006/011677
(87) International publication number: WO 2006/118719

(56) References cited:
- US-A1- 2002 044 957
- THOMA-WORRINGER ET AL: "Health effects and technological features of caseinomacropeptide" INTERNATIONAL DAIRY JOURNAL, ELSEVIER APPLIED SCIENCE, BARKING,, GB, vol. 16, no. 11, November 2006 (2006-11), pages 1324-1333, XP005671504 ISSN: 0958-6946
- WANG B ET AL: "Concentration and distribution of sialic acid in human milk and infant formulas" AMERICAN JOURNAL OF CLINICAL NUTRITION, BETHESDA,MD, US, vol. 74, 2001, pages 510-515, XP002388678 ISSN: 0002-9165
- WANG B ET AL: "Brain ganglioside and glycoprotein sialic acid in breastfed compared with formula-fed infants" AMERICAN JOURNAL OF CLINICAL NUTRITION 2003 UNITED STATES, vol. 78, no. 5, 2003, pages 1024-1029, XP002415483 ISSN: 0002-9165
- MANSO M A ET AL: "KAPPA-CASEIN MACROPEPTIDES FROM CHEESE WHEY: PHYSIOCHEMICAL, BIOLOGICAL, NUTRITIONAL AND TECHNOLOGICAL FEATURES FOR POSSIBLE USES" FOOD REVIEWS INTERNATIONAL, NEW YORK, NY, US, vol. 20, no. 4, 2004, pages 329-355, XP009068906
- WANG B ET AL: "The role and potential of sialic acid in human nutrition." EUROPEAN JOURNAL OF CLINICAL NUTRITION NOV 2003, vol. 57, no. 11, November 2003 (2003-11), pages 1351-1369, XP009077412 ISSN: 0954-3007
- RAHMANN HINRICH: "Brain gangliosides and memory formation" NATURWISSENSCHAFTEN, vol. 81, no. 1, 1994, pages 7-20, XP001248911 ISSN: 0028-1042
- BRODY E P: "Biological activities of bovine glycomacropeptide" BRITISH JOURNAL OF NUTRITION 2000 UNITED KINGDOM, vol. 84, no. SUPPL. 1, 2000, pages S39-S46, XP009077372 ISSN: 0007-1145

## Description

### BACKGROUND OF THE INVENTION

### (1) Field of the Invention:

The present invention relates to a method of improving learning and/or memory behavior in mammals by dietary means, and more particularly to a method of improving learning and/or memory behavior in a mammal by administration of a dietary source of sialic acid. (2) Description of the Related Art:

It is recognized that breast milk and breastfeeding is the preferred mode for feeding the human infant. Among the recognized benefits of breastfeeding is optimal mental development. For infants consuming infant formulas, there has been substantial effort to provide formula compositions that support optimal mental development when compared with that observed in the breastfed infant.

Compared with other primates, the adult human brain is four times larger relative to body mass, but the human infant is relatively immature at birth. Parker, S. T. et al., Origin of Intelligence, pp. 313 - 345, The Johns Hopkins Univ. Press, Baltimore, MD (1999). Brain growth, therefore, proceeds rapidly both before and after birth, placing enormous demands on the supply of precursor compounds required for brain development. Sub-optimal nutrition during a critical phase of early brain development may have long term effects on cognitive function - a matter of major public health and clinical concern. Lucas, A. et al., BMJ, 217:1481- 1487 (1998), and Lucas, A. et al., Lancet, 339:261-264 (1992). In a randomized, blinded, controlled trial in preterm infants, those fed a standard infant formula rather than nutrient-enriched formula had reduced verbal intelligence quotient (IQ) at 8 years of age. See, Lucas, *Id,* (1998). Disturbingly, almost half of the male infants fed standard formula had a sub-normal IQ compared with only 13% of those fed enriched milk. Such studies highlight the potential vulnerability of the increasing numbers of preterm and low birth weight infants to sub-optimal nutrition.

Specific components unique to human milk have the potential to support rapid brain growth. In particular, sialic acids (a family of N- and O-substituted derivatives of neuraminic acid; and in particular N-acetylneuraminic acid, or NANA) occur in large amounts as a component of human milk oligosaccharides (up to 1 g/l), and also forms the terminal functional residue of brain gangliosides and glycoproteins. Wang, B. et al., Am. J. Clin. Nutr., 74:510-515 (2001), and Carlson, S. E., Am J. Clin. Nutr., 41:720-726 (1985). Sialic acid components occur in highest concentrations in human milk just after birth. Idota et al., in J. of Japanese Soc. of Nutr. and Food Sci. (Nihon Eiyo Shokuryo Gakkai-shi), 47(5):363-367 (1994), show a dramatic decrease in the 6'-sialyllactose content of human breast milk from 3 to 482 days post partum.

In nature, the highest concentrations of sialic acid are found in the cerebral cortex of the human brain. Schauer, R., Sialic acids, Chemistry, Metabolism and Function, Springer-Verlag, Wien, New York (1982), and Svennerholm, L. et al., Biochem Biophys Acta, 1005:109-117 (1989). In particular, neural cell adhesion molecule (NCAM) is a sialydated protein that appears to play an important role in learning and memory. It is involved in a wide range of morphogenic events, including cell migration, neurite outgrowth, pathfinding, sprouting, regeneration and synaptic plasticity. Nakayama, J. et al., Virchows Archiv, 433:419-426 (1998), Mahal, L, K. et al., J. Biol. Chem., 277:9255-9261 (2002), and Ong, E. et al., Glycobiology, 8:415-424 (1998). In rodents, the degree of NCAM polysialylation is associated with increased learning and memory. Cremer, H. et al., Nature, 367:455-459 (1994).

Research has shown that the majority of N-acetylneuraminic acid (NANA) in the brains of rat pups administered NANA by intraperitoneal injection was incorporated into the synaptosomal fraction. Morgan, B. L. G. et al., Br. J. Nutr., 46:231-238 (1981). Later, Carlson, S. E., et al., in J. Nutr., 116:881-886 (1986) showed that both oral and intraperitoneal administration of N-acetylneuraminic acid resulted in significantly more cerebral and cerebellar ganglioside and glycoprotein N-acetylneuramic acid than did glucose injections.

Morgan, B. L. G. et al., in J. Nutr., 110:416-424 (1980), had also shown that malnourished rat pups that were injected intraperitoneally with N-acetylneuraminic acid learned a maze more quickly than litter mates injected with glucose, and showed reduced expected behavioral abnormalities due to malnutrition.

Cow's milk-based formulas generally have low sialic acid content. In one study, the concentration of sialic acid in several casein/whey combination formulas was less than 200 mg sialic acid/L. Moreover, soy protein-based formulas contain substantially reduced levels of sialic acid as compared to cow's milk-based formula. Therefore, formulas that are both lactose free and soy protein-based would exhibit very low sialic acid content. In some instances, research has shown that dietary supplementation with sialic acid, or with a sialic acid containing material provides certain benefits.

There are several known sources of sialic acid in its various conjugated forms. These include, but are not limited to, free N-acetylneuraminic acid (or sialic acid), the oligosaccharide sialyllactose, sialic acid-containing gangliosides, and the protein casein macropeptide (CMP), also referred to as glycomacropeptide (GMP), and, when obtained from cow's milk, casein glycomacropeptide (CGMP), or the like.

A method of producing CGMP is described in U. S. Patent Application 20040022918, which teaches that the manufacture of cheese from milk by coagulating cow's milk with rennet causes the coagulum to contract into a curd as it expresses whey. Casein macropeptide (CMP) is cleaved from the casein protein as a result of the action of the rennet on kappa casein and about 90% of the CMP is typically removed with the whey. CMP is a heterogeneous group of proteins, which contain all the genetic variations and post-translational modifications of kappa casein (Yvon et al., Reprod. Nutr. Dev., 34:527-537 (1994)). The predominant carbohydrate is sialic acid. Glycomacropeptide or GMP is the principal (50 to 75%) component of CMP. The carbohydrate content of the GMP renders it soluble in a 12% trichloroacetic acid solution. A number of analytical measurement techniques include a pre-treatment, which involves a TCA solution, this may remove at least a portion of the non-glycosylated CMP. For example the method published in The Official Journal of the European Communities (L228/10 Annex IV), details a HPLC method for measuring GMP in dairy products and uses the GMP level to calculate the level of cheese whey present in a sample. Other methods of producing CGMP from milk are described by Brody, E. P., in Br. J. of Nutr., 84(Suppl. 1):S39-S46 (2000).

The addition of sialic acid or sources of sialic acid to certain nutritional formulas has been discussed in U.S. Patent No. 6,506,422, which discloses a particular nutritional formula containing casein glycomacropeptide and complimentary essential amino acids other than phenylalanine for administration to patients suffering from phenylketonuria. The levels of sialic acid found in infant formulas are not mentioned.

U.S. Patent No. 6,270,827, discloses a formulation containing human milk proteins or recombinant host resistance factors, one of which is recombinant human kappa-casein, to supplement synthetic infant formulas.

U.S. Patent No. 4,762,822 discloses the use of N-acetylneuraminic acid or gangliosides containing sialic acid in infant formula to protect the newborn from gastrointestinal disease-producing organisms.

International patent application WO 01/60346 A2 discloses a nutritional formulation containing the oligosaccharides oligofructose and sialyllactose as prebiotic substances to promote the growth of bifidobacteria in the gut that may be used in conjunction with infant formula.

WO-A-00 49885 describes the use of a milk protein hydrolysate for addressing bone or dental disorders. Casein glycomacropeptide (CGMP) is extracted from sweet whey by a combination of electrodialysis, cation exchange resin, anion exchange resin, evaporation, spray drying, ultrafiltration and freeze drying, and is used to enrich foods or liquid enteral compositions.

Although the dietary administration of sialic acid has been reported for several purposes, dietary supplementation with sialic acid has not been shown to affect the learning or memory behavior of a mammal. Because dietary supplementation is an easy and widely accepted method of administering various agents to subjects, and in particular to infant mammals, it would be useful to provide a method by which the learning and memory behavior of a mammal could be improved by dietary means. Such a method would be particularly useful for neonatal mammal subjects that were in need of improvement in learning and memory behavior.

### SUMMARY OF THE INVENTION

Briefly, therefore the present invention is directed to a novel non-therapeutic method of improving learning and/or memory behavior in a human infant, the method comprising administering to the mammal casein glycomacropeptide in an amount sufficient to improve learning and/or memory behavior in the mammal,

Also described is a novel method of increasing brain protein-bound sialic acid in a mammal, the method comprising administering to the mammal an amount of casein glycomacropeptide that is sufficient to increase the brain protein-bound sialic acid in the mammal.

Also described is a novel method of improving learning and/or memory behavior in a mammal, the method comprising: determining whether the mammal is one that is in need of improvement in learning and/or memory; and, if so, administering to the mammal casein glycomacropeptide in an amount sufficient to improve learning and/or memory behavior in the mammal.

Among the several advantages found to be achieved by the present invention, therefore, may be noted the provision of a method by which the learning and/or memory behavior of a human infant is improved by wherein the amount sufficient to improve learning and/or memory provides at least 100mg/kg/day of sialic acid to the infant. dietary means, and also the provision of such a method that is particularly useful for neonatal mammal subjects that are in need of improvement in learning and memory behavior.

### BRIEF DESCRIPTION OF THE DRAWINGS

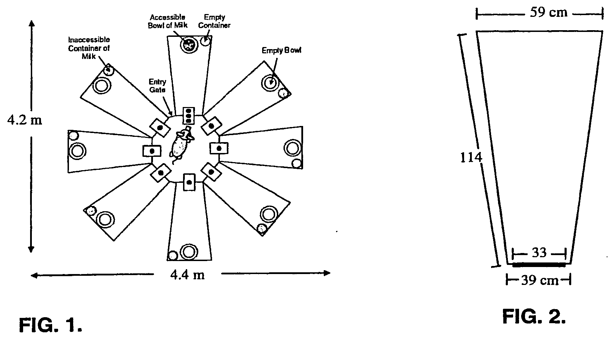

FIG. 1 is a schematic illustration of an 8-arm maze that is useful for testing learning and memory behavior in piglets;
FIG. 2 is a schematic illustration of one of the arms of the 8-arm maze shown in FIG. 1;

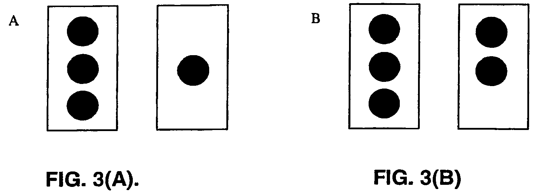

FIG. 3 shows visual cues used (A) in learning task 1, and (B) in learning task 2, in testing learning behavior of piglets;

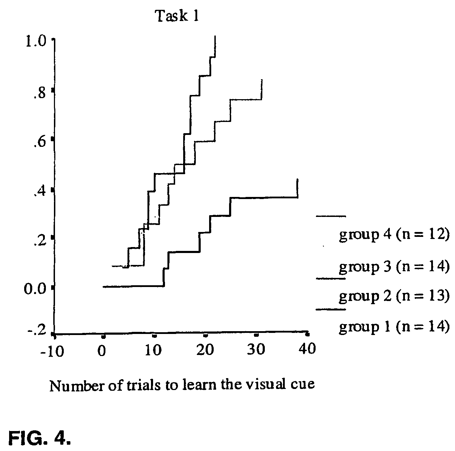

FIG.4 is a graph showing the fraction of piglets in each of four groups who learned the correct response to the visual cue in task 1 as a function of the number of trials, where Group 4 piglets (2^{nd} best performance) received a diet having 842 mg/L of sialic acid with supplementation by casein glycomacropeptide (240 mg/kg/day of sialic acid), Group 3 piglets (3^{rd} best) received a diet having 600 mg/L sialic acid with supplementation by casein glycomacropeptide (171 mg/kg/day of sialic acid), Group 2 piglets (best) received a diet having 250 mg/L sialic acid with supplementation from casein glycomacropeptide (71 mg/kg/day of sialic acid), and Group 1 piglets (worst performance) received a diet supplying 77 mg/L sialic acid (25 mg/kg/day of sialic acid) with no supplementation from casein glycomacropeptide;

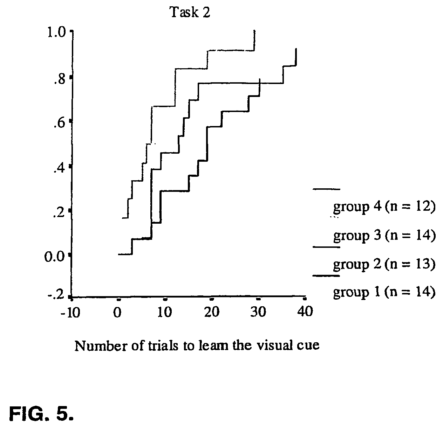

FIG. 5 is a graph showing the fraction of piglets in each of four groups who learned the correct response to the visual cue in task 2 as a function of the number of trials, where Group 4 piglets (best performance) received a diet having 842 mg/L of sialic acid with supplementation by casein glycomacropeptide (240 mg/kg/day of sialic acid), Group 3 piglets (2^{nd} best) received a diet having 600 mg/L sialic acid with supplementation by casein glycomacropeptide (171 mg/kg/day of sialic acid), Group 2 piglets (3^{rd} best) received a diet having 250 mg/L sialic acid with supplementation from casein glycomacropeptide (71 mg/kg/day of sialic acid), and Group 1 piglets (worst performance) received a diet supplying 77 mg/L sialic acid (25 mg/kg/day of sialic acid) with no supplementation from casein glycomacropeptide;

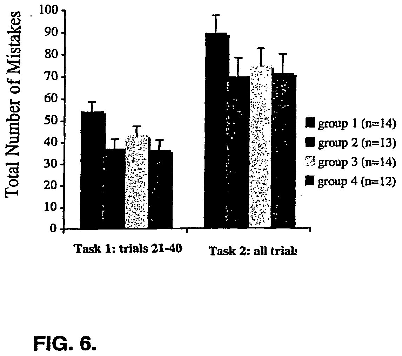

FIG. 6 is a bar graph showing the total number of mistakes by each group of piglets in tasks 1 and 2, and indicating that the highest total number of mistakes for each task was made by piglets in the group having no dietary supplementation with casein glycomacropeptide, and that supplementation at all levels improved the piglets learning ability;

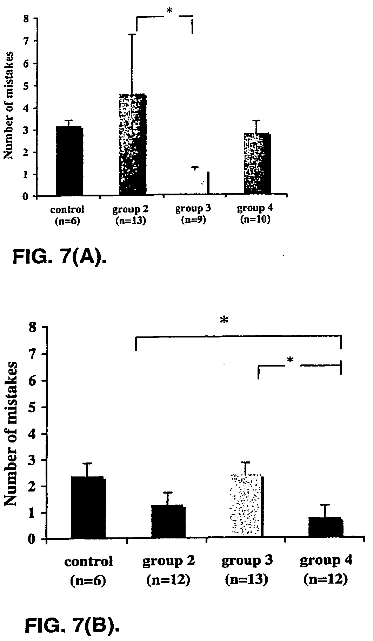

FIG. 7 shows two bar graphs, where graph (A) shows the mean number of mistakes made by piglets in each of the four groups for the memory test in task 1, and (B) shows the mean number of mistakes made by piglets in each of the four groups for the memory test in task 2;

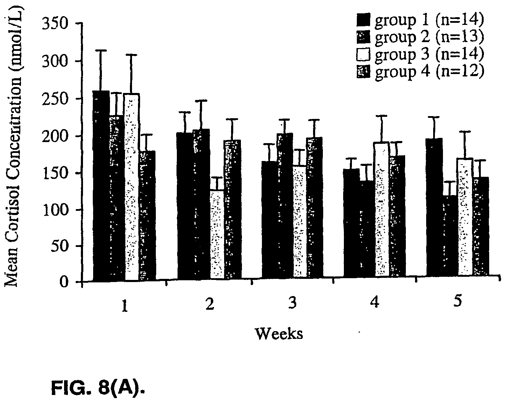

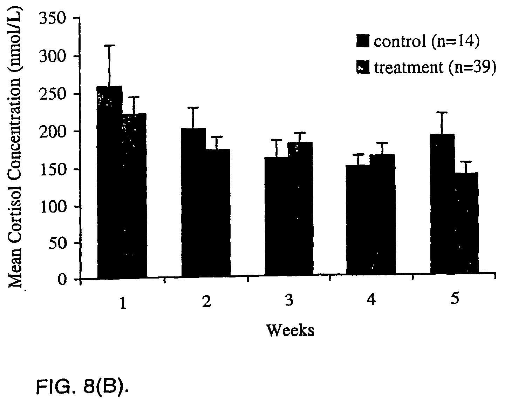

FIG. 8 shows two bar graphs, where (A) shows a comparison of the plasma cortisol levels between each of the four groups of piglets for each week of the study, and (B) shows a comparison of the plasma cortisol levels between combined treatment groups versus the control group for each week of the five week study;

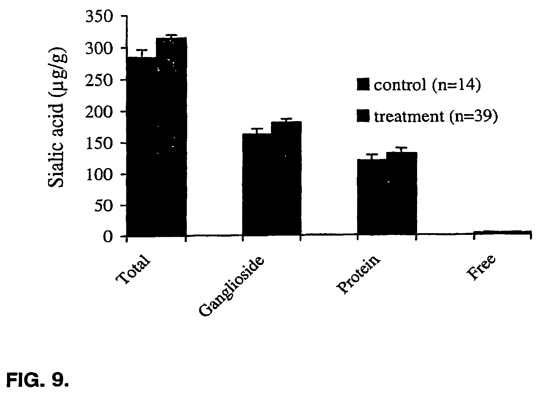

FIG. 9 is a bar graph that shows a comparison of the sialic acid concentration in brain grey matter between treatment groups and control, and which indicates higher sialic acid content in the brains of piglets receiving casein glycomacropeptide dietary supplementation;

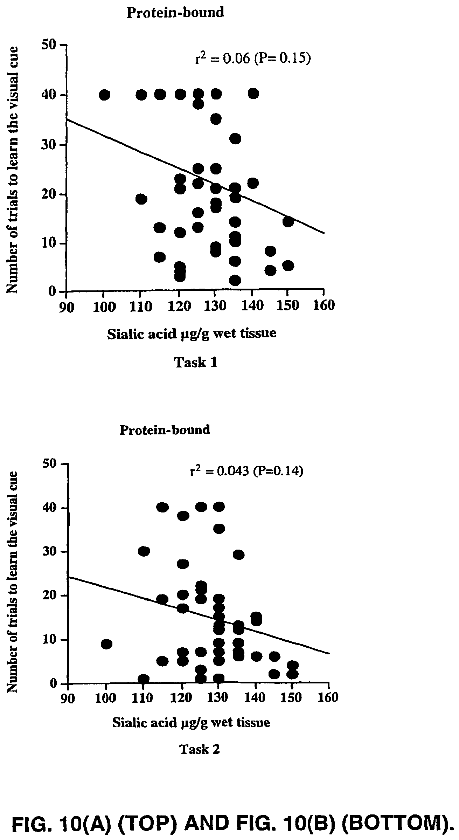

FIG. 10 shows two scatter charts of the concentration of protein-bound sialic acid in brain frontal cortex tissue versus the number of trials to learn the visual clue for (A) piglets in task 1, and (B) piglets in task 2, where both charts indicate that higher levels of protein-bound sialic acid in brain frontal cortex tissue correlate with improved learning behavior in piglets;

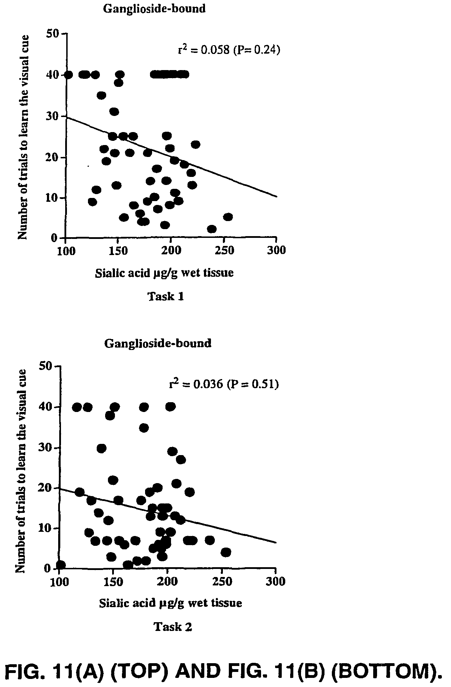

FIG. 11 shows two scatter charts of the concentration of ganglioside-bound sialic acid in brain frontal cortex tissue versus the number of trials to learn the visual clue for (A) piglets in task 1, and (B) piglets in task 2, where both charts indicate that higher levels of ganglioside-bound sialic acid in brain frontal cortex tissue correlate with improved learning behavior in piglets;

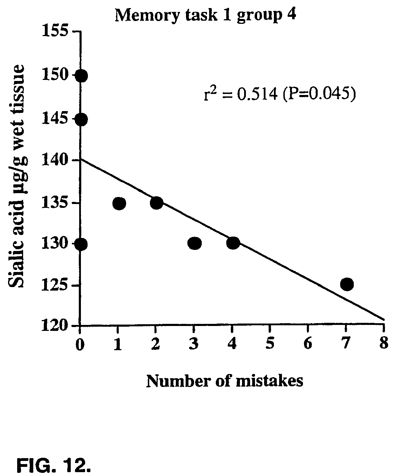

FIG. 12 shows a plot of the number of mistakes made in memory task 1 by group 4 piglets as a function of the concentration of protein-bound sialic acid in brain frontal cortex tissue, and indicates that having higher levels of protein-bound sialic acid in brain frontal cortex tissue make fewer mistakes in a memory test, and have improved memory behavior over those having lower levels of sialic acid; and

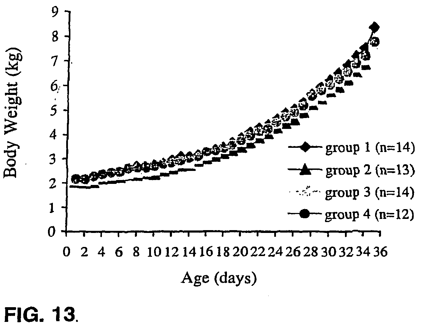

FIG. 13 is a chart showing mean body weight gain for the piglets in each of the four groups over the duration of the test, and which shows that the diet of each group was comparable with regard to meeting overall nutritional requirements of the piglets.

### DETAILED DESCRIPTION OF THE PREFERRED EMBODIMENTS

In accordance with the present invention, it has been discovered that leaning and/or memory behavior in a human infant can be improved by administering to the mammal casein glycomacropeptide in an amount sufficient to improve learning and/or memory behavior in the mammal. In a useful embodiment, the casein glycomacropeptide can be included in a formula.

It has now been shown that piglets fed diets containing 77 mg/L of sialic acid (no supplementation), and 250 mg/L, 600 mg/L, and 842 mg/L of sialic acid supplied by the supplementation of the diets with casein glycomacropeptide exhibited differences in both learning behavior and memory behavior. In fact, it was shown that both the learning behavior and the memory behavior were improved in piglets receiving the diets supplemented with casein glycomacropeptide, relative to the piglets receiving the non-supplemented, but otherwise nutritionally sufficient diets.

This improvement was unexpected, because the inventors were aware of no previous findings of improvement in learning and memory due to dietary supplementation of an otherwise nutritionally sufficient diet with casein glycomacropeptide. Also, prior studies had shown a correlation between reduced food intake and CGMP inclusion in the diet. See, e.g., U. S. Patent Publication Nos. 20040077530, and 20030059495, for example. Because reduced food intake is associated more often with lower mental acuity, rather than improved learning and memory, it was unexpected for dietary supplementation with CGMP to actually improve such performance, as was shown in the present invention.

The inventors have found that casein glycomacropeptide is a surprisingly good source for dietary supplementation to improve learning and memory behavior. It is surprising because cow's milk, in general, is low in sialic acid, and also, the macromolecules to which sialic acid is bound are different than those found in human milk, for example. Nonetheless, the inventors have found casein glycomacropeptide to be a surprisingly effective dietary supplement to improve learning and memory behavior, and furthermore, CGMP is relatively inexpensive, readily available, and nutritionally safe for infants.

The present invention is useful for any mammal. However, it is particularly useful for humans. The mammal can be of any age. However, it has been found that the present method is particularly useful when the mammal is between about 1 day and about 4 years of age. This age range is meant to include infants and toddlers when the mammal is a human. It is preferred that the mammal is a neonatal mammal. As used herein, the term "neonatal" is meant to describe the ages between birth and about two years.

The present method is particularly useful when the human infants is one that is in need of improvement in learning and/or memory behavior. When the terms "in need of improvement in learning and/or memory behavior" are used herein, they are meant to describe a subject who could benefit, no matter how greatly or slightly, from an improvement in learning behavior and/or memory behavior. The present method optionally includes the step of determining whether the human infant is one that is in need of an improvement is learning and/or memory; and, if so administering to the human infant a formula comprising casein glycomacropeptide in an effective amount.

The terms, "improvement in learning behaviour", are meant to include any improvement, no matter how slight, in the learning ability of the subject. The terms, "improvement in memory behavior", are meant to include any improvement, no matter how slight, in the memory behavior of the subject. The improvement in learning and/or memory behavior can be measured by any one or more of several tests that are useful for measuring learning and memory behavior in human infants. Examples of such tests include, without limitation, the Fagan Test of Infant Intelligence (FTII), the Dunst adaptation of the Uzgiris-Hunt Object Permanence Scale (OP), the Cross Modal Transfer (CMT) test, the Bayley Scales of Infant Development, Second Edition, Mental Development Index (MDI), the Bayley Infant Neurodevelopment Screener (BINS), and the Amiel-Tison Neurologic Exam (AT). Further information on testing of learning and memory in human infants can be found in Bayley, N., Manual for the Bayley scales of infant development, Psychological Corporation, New York (1969); Bates, J. E. et al., Child Dev., 50:794-803 (1979); and Black, M. M. et al., Bayley Scales of Infant Development II Assessment, Unlimited Learning Resources, Winston-Salem, NC (2003).

It is believed that a need of improvement in learning and/or memory behavior can be caused in a, human infant, by any of a number of factors that are known in the art. By way of example, malnutrition, the presence of environmental factors - such as ingestion of certain metals, deprivation of oxygen, trauma, disease, and other factors, can cause a need for such improvement. In one situation, as discussed above, the prior art has inferred some relationship between the cognitive ability of some neonatal mammals and the dietary level of certain nutrients - and sialic acid has been shown to be one of these nutrients. Accordingly, it is believed that in the present invention the need of improvement in learning and/or memory behavior is shown, or the determination can be made that the mammal is one that is in need of improvement in learning and/or memory, where at least a portion of the mammal's nutritional requirement has been supplied by a formula having less than 100 mg/L of sialic acid, and in particular, where substantially all of the mammal's nutritional requirement has been supplied by administration of a formula having less than 100 mg/L of sialic acid.

When it is said that "at least a portion of the human infant's nutritional requirement has been supplied" by a certain formula, it is meant that at least 25% of the human infant's nutritional requirements has been supplied for at least a majority of the period from birth to the present age of the human infant by the formula. It is preferred that the portion of the human infant's nutritional requirements that has been supplied for at least a majority of the period from birth to the present age of the human infant is at least 50%, more preferred is 75%, and yet more preferred is substantially all of the human infant's nutritional requirements.

Due to the low level of sialic acid in soy protein and cow's milk protein, as compared with the sialic acid levels in human milk - especially in colostrum and milk produced during early lactation, it is believed that a mammal receiving a formula wherein the major part of the protein is soy protein or cow's milk protein, and in which the level of sialic acid is less than about 100 mg/L, is one that is in need of improvement in learning and/or memory behavior. In particular, this is believed to be the case when at least about 75% by weight of the protein contained in the formula is soy protein or cow's milk protein, and is yet is more true when substantially all of the protein contained in the formula is soy protein or cow's milk protein.

In fact, it is believed that the need of improvement in learning and/or memory behavior in a human infant is shown where the mammal's nutritional requirement has been supplied by a diet providing sialic acid in an amount that is lower than would normally be obtained from breastfeeding. When it is said that "the infant's nutritional requirement has been supplied", it is meant that the infant's nutritional requirement has been supplied for at least a majority of the period from birth to the present age of the mammal. The terms "a diet providing sialic acid in an amount that is lower than would normally be obtained from breastfeeding", means a diet comprising a liquid formula having a sialic acid content that is below 100 mg/L. In other embodiments, and in some embodiments, a liquid formula having a sialic acid content that is below 200 mg/L.

In the present invention, the formula that comprises casein glycomacropeptide can be administered to the human infant by any type of enteral administration. As used herein, enteral administration includes administration of a formula to any point in the GI tract of the infant, and includes without limitation, oral administration, and enteral tubular administration.

Although the casein glycomacropeptide can be administered to a a human infant as is, and without any accompanying compounds or materials, it is useful to provide the CGMP as one ingredient of a formula. The formula that comprises casein glycomacropeptide can be any nutritional formula, but is preferably an infant formula. In some embodiments, the infant formula is a nutritionally complete infant formula comprising carbohydrate, lipid, and protein. The infant formula for use in the present invention can be nutritionally complete, or it can be a supplemental formula. Typically, the formula contains suitable types and amounts of lipids, carbohydrates, proteins, vitamins and minerals. The amount of lipids or fats typically can vary from 3 to 7 g/100 kcal. The amount of proteins typically can vary from 1 to 5g/100 kcal. The amount of carbohydrates typically can vary from 6 to 16 g/100 kcal.

As used herein, the term "formula" means a man-made composition, and is not to be interpreted to include breast milk, for example.

Protein sources can be any used in the art, and may include, for example, nonfat milk, whey protein, casein, soy protein, hydrolyzed protein, and amino acids. Lipid sources can be any used in the art such as, for example, vegetable oils such as palm oil, soybean oil, palm olein oil, com oil, canola oil, coconut oil, medium chain triglyceride oils, high oleic sunflower oil, and high oleic safflower oil. Carbohydrate sources can be any known in the art such as, for example, lactose, glucose polymers, corn syrup solids, maltodextrins, sucrose, starch, and rice syrup solids.

Conveniently, several commercially available infant formulas can be used as the basic formula for the CGMP additions. For example, Enfamil® Lipil with iron (available from Mead Johnson & Company, Evansville, Indiana, U.S.A.) may be supplemented with an effective amount of CGMP and used to practice the method of the present invention. Particular infant formulas suitable for use in the present invention are described in the Examples herein.

The total protein in the formulation from all protein sources should be nutritionally appropriate for infants, which is typically from 12 g per liter to 18 g per liter and, in some embodiments, may be 14 g per liter. The total sialic acid in the formulation may be between 200 and 1500 mg per liter. It is preferred that the present formula comprises a liquid having a sialic acid concentration of at least mg/liter. In some embodiments, it is more preferred that the formula comprises a liquid having a sialic acid level of at least 300 mg/liter, and a sialic acid level of at least 600 mg/liter is yet more preferred. It is preferred that the formula contains up to 6 g per liter of casein glycomacropeptide (CGMP) or related protein fraction, as commercially available from various sources, containing 81% protein and between 40 and 300 mg sialic acid per gram of protein, but typically between 40 and 60 mg SA/gm protein, and, thus, contributing between 194 and 1458 mg sialic acid per liter of formula, but typically between 194 and 290 mg SA/liter of formula; or up to 6 g/liter of a CGMP fraction having an enhanced level of sialic acid.

The casein glycomacropeptide that is useful in the present invention, in general, can be from any source and of any purity or grade that is suitable for nutritional use, or for inclusion in an infant formula. Casein glycomacropeptide may be extracted from milk using suitable processing. For example, the casein glycomacropeptide may be extracted from the retentate obtained from the concentration of whey protein. This may be done by at least partially removing lactose from the retentate and then adding ethanol to cause precipitation. The supernatant is then collected and dried to provide the casein glycomacropeptide. U.S. Patent No. 5,216,129 provides a more detailed description of this process. CGMP that is useful in the present method can also be produced according to the techniques described in U.S. Patent Nos. 6,555,659, 5,280,107, 5,968,586, and 5,075,424, and in PCT/US94/15952, and WO 03/049547. Alternatively, the CGMP may be purchased from commercial sources such as, for example, The Tatua Co-Operative Dairy Company Limited, Tatuanui, Morrinsville, New Zealand, MD Foods Ingredients amba of DK-6920 Videbaek, Denmark or from DMV International of NCB-laan 80, NL-5460 BA Veghel, The Netherlands.

In the present method, it is preferred that the casein glycomacropeptide, or the formula comprising casein glycomacropeptide, is administered in an amount sufficient to provide 100 mg/kg/day of sialic acid to the mammal, and in some embodiments, the provision of 200 mg/kg/day of sialic acid to the mammal is more preferred.

In an embodiment of the present invention, it is preferred that the formula has total protein content of between 12 and 16 grams/liter of which no more than 40% by weight is provided by casein glycomacropeptide. It is more preferred that the formula comprises a total protein content of between 13 and 15 grams/liter of which no more than 30% by weight is provided by casein glycomacropeptide. In one embodiment, the protein content of the formula is provided by casein glycomacropeptide and soy protein.

Some embodiments of the present invention involve the use of novel CGMP products that contain levels of sialic acid that are higher than normally found in standard CGMP products that are commercially available. These novel products can be used alone or in combination to achieve sialic acid levels that mimic those found in breast milk, based on the sialic acid content of the various source ingredients. In one embodiment, the casein glycomacropeptide comprises a casein glycomacropeptide having an enhanced concentration of sialic acid.

As used herein, the terms "CGMP having an enhanced concentration of sialic acid" mean a casein glycomacropeptide (CGMP)-containing fraction of milk that has been treated to increase the level of sialic acid, and in which the level of sialic acid is higher, by any amount, than before the treatment. CGMP products with enhanced levels of sialic acid are described below in Reference Examples 2 and 3.

One such product, an example of which is described in Reference Example 2, can be referred to herein as "CGMP having an enhanced level of sialic acid", or "high-sialic acid CGMP". High-sialic acid CGMP has a sialic acid content of above about 60 mg/gm protein. It is preferred that the sialic acid content is above about 100 mg/gm protein, more preferred is above about 150 mg/gm protein, and yet more preferred is a sialic acid content of above 200 mg/gm protein. Typically, this product has a protein content of 50% - 60% by weight for a dry powder product, a sialic acid content of 190 - 230 mg/gm protein, or about 100-130 mg/gm powder. In comparison, regular CGMP dry powder (for example, glycomacropeptide available from Tatua Co-Operative Dairy Company Limited) contains 81% protein by weight, and has a sialic acid content of 52 mg/gm protein, or 42 mg/gm powder. It is apparent, therefore, that the sialic acid content of the high-sialic acid CGMP has been enhanced over that of the regular glycomacropeptide powder by about 3-fold on the basis of powder weight, and 4-fold on the basis of protein content of the products. For comparison purposes, electrodialyzed (ED) whey powder contains 14% protein on a dry basis, and contains 30 mg of sialic acid/gm protein, or 4.3 mg of sialic acid/gm of powder.

An advantage of using a high-sialic acid CGMP as a protein source in an infant formula is that the sialic acid content of the formula can be increased without replacing an undue amount of the conventional sources of protein that are used in the formula. This feature is useful in that it permits minimal disruption of the amino acid profile of the protein of the formula.

In a particular embodiment of a high-sialic acid CGMP, the product has a level of the amino acid threonine that is lower than the level of that amino acid in the glycomacropeptide from which the novel product is derived. As used herein, this type of high-sialic acid CGMP is referred to as "CGMP having an enhanced level of sialic acid and reduced threonine", or "high-sialic acid CGMP with reduced threonine". An example of this type of product is described below in Reference Example 3.

High-sialic acid CGMP with reduced threonine has a sialic acid content of above 60 mg/gm protein and a threonine concentration that is lower than 15 gm/16 gm nitrogen. It is preferred that the sialic acid content is above 100 mg/gm protein, more preferred is above 150 mg/gm protein, and yet more preferred is a sialic acid content of above 200 mg/gm protein. Typically, high-sialic acid CGMP with reduced threonine can have a sialic acid content of from 85 to 150 mg sialic acid (SA)/ gram of powder, preferably from 90 to 140 mg SA/g powder, which is comparable to the sialic acid content of high-sialic acid CGMP. However, the threonine content of high-sialic acid CGMP with reduced threonine is only about one-fourth that of a commercial CGMP product. Preferably, the threonine content is below about 10 g/16 g nitrogen, more preferably below 7 gm/16 gm nitrogen, even more preferably below 5 g/16 g nitrogen, and yet more preferably below 4 g/16 g nitrogen. Expressed in an alternative manner, the threonine content is below 8% by weight of the total weight of amino acids of the protein, preferably below 6%, more preferably below 4%, and yet more preferably below 3%.

An advantage provided by this type of enhanced sialic acid product is that in addition to the increase in sialic acid with reduced amino acid profile disruption, as discussed above, the threonine level of the protein sources in the infant formula can be controlled. This is desirable in some embodiments in order to reduce or eliminate the potential for hyperthreoninuria, or other disorder caused by, or exacerbated by, high levels of threonine in the diet.

By way of example, an infant formula that is useful in the present invention can be formulated to have a sialic acid content of at least 200 mg/liter and have a total protein content of between 12 and 16 grams/liter of which no more than 40% by weight is provided by a CGMP having an enhanced concentration of sialic acid. Preferably, such an infant formula has a total protein content of between 13 and 15 grams/liter of which no more than 30% by weight is provided by a CGMP having an enhanced concentration of sialic acid, more preferably, the infant formula has a total protein content of between 13 and 15 grams/liter of which no more than 15% by weight is provided by a CGMP having an enhanced concentration of sialic acid.

Also as an example, an infant formula that is useful in the present invention can be formulated to have a sialic acid content of at least 400 mg/liter and have a total protein content of between 13 and 15 grams/liter of which no more than 15% by weight is provided by a CGMP having an enhanced concentration of sialic acid.

The casein glycomacropeptide-supplemented formulas that are useful in the present invention can be used in the same manner as any other commercial infant formula. It can be produced in powder form, for later reconstitution with a liquid, or it can be prepared in liquid form. The formula should be packaged, stored, handled, and distributed in the same manner as any other similar product, and should, in general, be used in the same fashion.

The following examples describe exemplary embodiments of the invention. It is intended that the specification, together with the examples, be considered to be exemplary only. In the examples all percentages are given on a weight basis unless otherwise indicated.

### REFERENCE EXAMPLE 1.

This example illustrates the nutrient components in a commercial infant formula suitable for sialic acid addition for use in the present invention.

**Table 1: Nutrient Information for Infant Formula (Enfamil® Lipil with Iron)**

| NUTRIENTS (Normal Dilution) | Per 100 Calories (5 fl oz) |
|---|---|
| Protein, g | 2.1 |
| Fat, g | 5.3 |
| Carbohydrate, g | 10.9 |
| Water, g | 134 |
| Linoleic acid, mg | 860 |

| Vitamins: | |
|---|---|
| A, IU | 300 |
| D, IU | 60 |
| E, IU | 2 |
| K, µg | 8 |
| Thiamin (Vitamin B1), µg | 80 |
| Riboflavin (Vitamin B2), µg | 140 |
| B6, µg | 60 |
| B12, µg | 0.3 |
| Niacin, µg | 1000 |
| Folic acid (Folacin), µg | 16 |
| Pantothenic acid, µg | 500 |
| Biotin, µg | 3 |
| C (Ascorbic acid), mg | 12 |
| Choline, mg | 12 |
| Inositol, mg | 6 |

| Minerals: | |
|---|---|
| Calcium, mg | 78 |
| Phosphorus, mg | 53 |
| Magnesium, mg | 8 |
| Iron, mg | 1.8 |
| Zinc, mg | 1 |
| Manganese, µg | 15 |
| Copper, µg | 75 |
| Iodine, µg | 10 |
| Selenium, µg | 2.8 |
| Sodium, mg | 27 |
| Potassium, mg | 108 |
| Chloride, mg | 63 |

The ingredients of this particular formula are: reduced minerals whey, nonfat milk, vegetable oil (palm olein, soy, coconut, and high oleic sunflower oils), lactose, and less than 1%: mortierella alpina oil, crypthecodinium cohnii oil, vitamin A palmitate, vitamin D³, vitamin E acetate, vitamin K¹, thiamin hydrochloride, vitamin B6 hydrochloride, vitamin B¹², niacinamide, folic acid, calcium pantothenate, biotin, sodium ascorbate, inositol, calcium chloride, calcium phosphate, ferrous sulfate, zinc sulfate, manganese sulfate, cupric sulfate, sodium chloride, sodium citrate, potassium citrate, potassium hydroxide, sodium selenite, taurine, nucleotides (adenosine 5'-monophosphate, cytidine 5'-monophosphate, disodium guanosine 5'-monophosphate, disodium uridine 5'-monophosphate).

To use this particular formula to practice the present invention, it would be necessary to add, for example, casein glycomacropeptide to the formula in an amount sufficient to provide from about 250 mg per liter to about 1500 mg per liter of sialic acid to the composition described in Table 1. This added amount of sialic acid would be part of the total amount of protein (total protein of approximately 2.1 grams per 100 calories).

### EXAMPLE 1.

This example illustrates a particular protein source combination for a total sialic acid content of approximately 250 mg per liter. The ingredients listed in Table 2 would be used to replace the protein component of the formula described in Table 1.

**Table 2: Protein Source Composition A**

| Ingredient | mg SA/ gm protein^{a} | %of protein in ingredient | g ingredient/ L | g protein/ L | mg SA/ L |
|---|---|---|---|---|---|
| Whey Protein Concentrate | 23.00 | 35.00 | 20.26 | 7.09 | 163.08 |
| Nonfat Dry Milk, Low Heat | 6.37 | 34.00 | 15.38 | 5.23 | 33.31 |
| CGMP^{b} | 52.00 | 81.00 | 1.45 | 1.17 | 61.07 |

| | | | | | |
|---|---|---|---|---|---|
| Note: a. "SA" in table means sialic acid. b. CGMP means casein glycomacropeptide. | | | | | |

### EXAMPLE 2.

This example illustrates a particular protein source combination for a total sialic acid content of approximately 360 mg per liter. The ingredients listed in Table 3 replace the protein component of the formula described in Table 1.

**Table 3: Protein Source Composition B**

| Ingredient | mg SA/ gm protein^{a} | % of protein in ingredient | g ingredient/ L | g protein/ L | mg SA/ L |
|---|---|---|---|---|---|
| Whey Protein Concentrate | 23.00 | 35.00 | 37.00 | 12.95 | 297.85 |
| CGMP^{b} | 52.00 | 81.00 | 1.45 | 1.17 | 61.07 |

| | | | | | |
|---|---|---|---|---|---|
| Note: a. "SA" in table means sialic acid. b. CGMP means casein glycomacropeptide. | | | | | |

### EXAMPLE 3.

This example illustrates a particular protein source combination for a total sialic acid content of approximately 600 mg per liter. The ingredients listed in Table 4 replace the protein component of the formula described in Table 1.

**Table 4: Protein Source Composition B**

| Ingredient | mg SA/ gm protein^{a} | % of protein in ingredient | g ingredient/ L | g protein/ L | mg SA/ L |
|---|---|---|---|---|---|
| Whey Protein Concentrate | 23.00 | 35.00 | 13.00 | 4.55 | 104.65 |
| CGMP^{b} | 52.00 | 81.00 | 12.00 | 9.72 | 505.44 |

| | | | | | |
|---|---|---|---|---|---|
| Note: a. "SA" in table means sialic acid. b. CGMP means casein glycomacropeptide. | | | | | |

### EXAMPLE 4.

Table 5 illustrates an example of a complete nutritional formulation of an infant formula with a total sialic acid content of approximately 250 mg per liter.

**Table 5: Exemplary infant formulation with sialic acid.**

| Ingredient | Weight | Amount per 10000 liters |
|---|---|---|
| Lactose (95% Solids) | | 573.000 kg |
| Fat Blend | | 332.500 kg |
| Whey Protein Concentrate (36% Protein, 5.8% Ash) | | 202.578 kg |
| Nonfat Milk Solid (36% Prot., 52% CHO) | | 153.844 kg |
| Casein glycomacropeptide (CGMP, 81.18% Prot.) | | 14.500 kg |
| Mono-and Diglycerides | | 7.233 kg |
| Calcium Phosphate, Tribasic | | 6.520 kg |
| Single Cell Arachidonic Acid Oil | | 6.485 kg |
| Dry Vitamin Premix for Enfamil AR Liquid | | 5.250 kg |
| Ascorbic Acid | 2924.250g | |
| Inositol | 834.750g | |
| Corn Syrup Solid | 654.938g | |
| Taurine | 582.750g | |
| Niacinamide | 119.438g | |
| Calcium Pantothenate | 44.730g | |
| Vitamin B12, 0.1% in Starch | 29.400g | |
| Biotin, 1 % Trituration | 25.095g | |
| Thiamine Hydroxhloride | 13.913g | |
| Riboflavin | 10.238g | |
| Pyridoxine Hydrochloride | 8.138g | |
| Folic Acid | 2.363g | |
| Lecithin Concentrate | | 3.694 kg |
| Potassium Citrate | | 3.350 kg |
| Single Cell Docosahexaenoic Acid Oil | | 3.243 kg |
| Nucleotide Premix for Enfamil Powder | | 2.900 kg |
| Maltodextrin, 15 DE | 2552.290g | |
| Cytidine 5'-monophosphate, free acid | 202.710g | |
| Uridine 5'-monophosphate, disodium salt | 59.740g | |
| Adenosine 5'-monophosphate, free acid | 47.357g | |
| Guanosine 5'-monophosphate, disodium salt | 37.903g | |
| Carrageenan | | 2.826 kg |
| Magnesium Chloride | | 1.657 kg |
| Calcium Chloride, Dihydrate | | 1.200 kg |
| Choline Chloride | | 0.700 kg |
| Ferrous Sulfate Heptahydrate | | 0.682 kg |
| Sodium Citrate, Dihydrate, Granular | | 0.455 kg |
| Trace Mineral Premix w/Selenite Trituration | | 0.392 kg |
| Zinc Sulfate, Monohydate | 276.238g | |
| Sodium Selenite Trituration, 0.5% | 65.907g | |
| Cupric Sulfate, powder | 29.510g | |
| Lactose, Grind A | 16.323g | |
| Manganese Sulfate, monohydrate | 4.022g | |
| Vitamin A,D,E,K Premix, Enfamil Liquid | | 0.324 kg |
| Tocopherol Acetate | 160.882g | |
| Soybean Oil | 139.612g | |
| Vitamin A Palmitate | 17.253g | |
| Cholecalciferol Concentrate | 5.715g | |
| Vitamin K1, Liquid | 0.538g | |
| Ascorbic Acid | | 0.150 kg |
| L-Carnitine | | 0.150 kg |
| Water, Defluoridated, q.s. to | | 10310.986 kg |
| Potassium Hydroxide | | --- |

Table 6 and Table 7 show the content of specific components of the formulation described in Table 5 as a percentage of 1) weight to weight, 2) weight to volume, and 3) calories. The specific gravity of this particular formulation is 1.0310986.

**Table 6: Infant formulation composition.**

| Component | % w/w | % w/v |
|---|---|---|
| Protein | 1.38 | 1.42 |
| Fat | 3.50 | 3.61 |
| Carbohydrate | 7.20 | 7.43 |
| Ash | 0.37 | 0.38 |
| Total Solids | 12.45 | 12.84 |

**Table 7: Infant formula caloric distribution**

| Component | % |
|---|---|
| Protein | 8.38 |
| Fat | 47.83 |
| Carbohydrate | 43.79 |

### EXAMPLE 5.

Table 8 illustrates the nutritional content of the formulation presented in Example 4 per 100 calories, as well as per 100 milliliters of formula.

**Table 8: Nutritional content of infant formulation.**

| | Per 100 Cal | Per 100 ml |
|---|---|---|
| Calories, Cal | 100 | 68 |
| Protein, g | 2.1 | 1.42 |
| Fat, g | 5.3 | 3.6 |
| Carbohydrate, g | 10.9 | 7.4 |
| Linoleic Acid, mg | 860 | 580 |
| Linolenic Acid, mg | 80 | 54 |
| Arachidonic Acid, mg | 34 | 23 |
| Docosahexaenoic Acid, mg | 17 | 11.5 |
| | | |
| Vitamin A, IU | 300 | 200 |
| Vitamin D, IU | 60 | 41 |
| Vitamin E, IU | 2 | 1.35 |
| Vitamin K1, mcg | 12 | 8.1 |
| Thiamin, mcg | 120 | 81 |
| Riboflavin, mcg | 140 | 95 |
| Vitamin B6, mcg | 60 | 41 |
| Vitamin B12, mcg | 0.5 | 0.3 |
| Niacin, mcg | 1200 | 812 |
| Folic Acid, mcg | 16 | 10.8 |
| Pantothenic Acid, mcg | 500 | 340 |
| Biotin, mcg | 3 | 2 |
| Vitamin C, mg | 14 | 9.5 |
| Choline, mg | 12 | 8.1 |
| Inositol, mg | 6 | 4.1 |
| Taurine, mg | 6 | 4.1 |
| L-Carnitine, mg | 2 | 1.35 |
| | | |
| Calcium, mg | 78 | 53 |
| Phosphorus, mg | 53 | 36 |
| Magnesium, mg | 8 | 5.4 |
| Iron, mg | 1.8 | 1.2 |
| Zinc, mg | 1 | 0.68 |
| Manganese, mcg | 26 | 17.6 |
| Copper, mcg | 85 | 57 |
| Iodine, mcg | 15 | 10 |
| Sodium, mg | 27 | 18.3 |
| Potassium, mg | 108 | 73 |
| Chloride, mg | 63 | 43 |
| Selenium, mcg | 2.8 | 1.89 |
| Sialic acid, mg | 37 | 25 |
| | | |
| Calcium/Phosphorus Ratio | --- | --- |
| | | |
| AMP Equivalents, mg (a) | 0.5 | 0.34 |
| CMP Equivalents, mg (a) | 2.5 | 1.69 |
| GMP Equivalents, mg (a) | 0.3 | 0.20 |
| UMP Equivalents, mg (a) | 0.9 | 0.61 |
| Nucleotide Equivalents, mg (a) | 4.2 | 2.84 |
| TPAN-AMP, mg | -- | -- |
| TPAN-CMP, mg | -- | -- |
| TPAN-GMP, mg | -- | -- |
| TPAN-UMP, mg | -- | -- |
| Total TPAN, mg | -- | -- |
| TPAN-CMP/TPAN-GMP Ratio | -- | -- |

| | | |
|---|---|---|
| Note: Sum of the nucleotide and corresponding nucleoside expressed as the nucleotide weights. | | |

### REFERENCE EXAMPLE 2.

This illustrates the production of a CGMP product having enhanced levels of sialic acid.

A fraction of cheese whey that is enriched in GMP is fractionated by using anion chromatography to yield a fraction that is enhanced in sialic acid. This product exhibits an amino acid profile similar to that of currently commercially available GMP (available from Tatua Co-Operative Dairy Company Limited, Tatuanui, Morrinsville, New Zealand), but contains from 1.5 - 3 times the sialic acid content of currently available GMP products.

The sialic acid-enhanced fraction can be desalted, if desired, by electrodialysis, for example, and can be dried to yield a dry powder product, which is then useable for introduction into a liquid or a dry infant formula mix. This product is a high-sialic acid CGMP and is available as of the filing date of the present application from Tatua Co-Operative Dairy Company Limited, as products designated as X4738, X4739, X4740, and X4741. The protein content, sialic acid content, and amino acid profile of those materials is described in Table 9.

**Table 9: Amino acid profile and sialic acid content of four examples of high-sialic acid CGMP products.**

| Amino Acid | High-Sialic Acid CGMP Product Samples | | | | | | |
|---|---|---|---|---|---|---|---|
| | X4738 | X4739 | X4740 | X4741 | Average | CGMP Powder | ED Whey Powder |
| Arginine | 1.22 | 0.96 | 0.69 | 0.7 | 0.89 | 1.4 | |
| Histidine | 0.76 | 0.7 | 0.59 | 0.59 | 0.66 | 1 | |
| Isoleucine | 10.36 | 8.42 | 11.28 | 11.51 | 10.39 | 11.6 | |
| Leucine | 4 | 3.36 | 3.19 | 3.21 | 3.44 | 4.6 | |
| Lysine | 7.48 | 7.91 | 6.89 | 7.08 | 7.34 | 8.3 | |
| Methionine | 2 | 2.04 | 1.63 | 1.63 | 1.83 | 1.5 | |
| Cystine | 0.21 | 0.47 | 0.13 | 0.09 | 0.23 | 0.2 | |
| phenylalanine | 1.54 | 5.88 | 1.96 | 2.08 | 2.87 | 1.8 | |
| Tyrosine | 0.35 | 0.17 | 0.1 | 0.09 | 0.18 | 0.4 | |
| Threonine | 13.13 | 15.16 | 17.18 | 17.57 | 15.76 | 15.9 | |
| Tryptophan | 0 | 0 | 0 | 0 | 0.00 | 0 | |
| Valine | 8.69 | 7.55 | 9.38 | 9.51 | 8.78 | 9.7 | |
| Alanine | 6.8 | 6.58 | 6.41 | 6.53 | 6.58 | 6.6 | |
| aspartic acid | 10.61 | 12.12 | 9.93 | 10 | 10.67 | 11.1 | |
| glutamic acid | 22.91 | 24.23 | 23.28 | 23.14 | 23.39 | 26.2 | |
| Glycine | 1.37 | 1.46 | 1.31 | 1.34 | 1.37 | 1.5 | |
| Proline | 11.13 | 10.19 | 10.78 | 9.57 | 10.42 | 14.2 | |
| Serine | 8.14 | 9.66 | 8.74 | 9.06 | 8.90 | 8.1 | |
| | | | | | | | |
| TOTAL | 110.7 | 116.86 | 113.47 | 113.7 | 113.68 | 124.1 | |
| | | | | | | | |
| | | | | | | | |
| % protein | 51.88 | 49.92 | 57.87 | 60.05 | 54.93 | 81 | 14.31 |
| mgSA/gm protein | 188.43 | 227.25 | 224.83 | 215.68 | 214.05 | 52 | 29.92 |
| mgSA/gm powder | 97.76 | 113.44 | 130.11 | 129.52 | 117.71 | 42.12 | 4.28 |
| Average | | | | | | | |
| | | | | | | | |
| Amino acid levels are expressed as grams of the amino acid per 16 grams of nitrogen. | | | | | | | |
| CGMP Powder is commercial glycomacropeptide from Tatua Co-Operative Dairy Company Ltd. | | | | | | | |
| ED Whey Powder is commercial electrodialyzed whey powder | | | | | | | |
| Samples X4738 - X4741 are samples of high-sialic acid CGMP available from Tatua Co-Operative Dairy Company Limited, Tatuanui, Morrinsville, New Zealand. | | | | | | | |

### REFERENCE EXAMPLE 3.

This illustrates the production of a CGMP product having enhanced levels of sialic acid and low levels of threonine.

A fraction of cheese whey that is enriched in GMP is subjected to a partial proteolytic hydrolysis followed by fractionation by using anion chromatography to yield a fraction that is enhanced in sialic acid and has a low threonine content. This product contains from 1.5 - 3 times the sialic acid content of currently available GMP products, but the level of threonine is reduced to about one-fourth that of the starting GMP material.

The sialic acid-enhanced, low threonine fraction can be desalted, if desired, by electrodialysis, for example, and can be dried to yield a dry powder product, which is then useable for introduction into a liquid or a dry infant formula mix. This product is a high-sialic acid CGMP with reduced threonine and is available as of the filing date of the present application from Tatua Co-Operative Dairy Company Limited, Tatuanui, Morrisnville, New Zealand, as product designated as W4733. The protein content, sialic acid content, and amino acid profile of that material is described in Table 10.

**Table 10: Amino acid profile and sialic acid content of high-sialic acid CGMP with reduced threonine.**

| Amino Acid | High-Sialic Acid CGMP with Reduced Threonine | | |
|---|---|---|---|
| | W4733 | CGMP Powder | ED Whey Powder |
| arginine | 2.3 | 1.4 | |
| histidine | 0 | 1 | |
| isoleucine | 13.1 | 11.6 | |
| leucine | 5.3 | 4.6 | |
| lysine | 3.2 | 8.3 | |
| methionine | 0.7 | 1.5 | |
| cystine | 0.1 | 0.2 | |
| phenylalanine | 0 | 1.8 | |
| tyrosine | 0 | 0.4 | |
| threonine | 3.8 | 15.9 | |
| tryptophan | 0 | 0 | |
| valine | 16.3 | 9.7 | |
| alanine | 15.9 | 6.6 | |
| aspartic acid | 6.3 | 11.1 | |
| glutamic acid | 38.9 | 26.2 | |
| glycine | 2.5 | 1.5 | |
| proline | 16.9 | 14.2 | |
| serine | 0 | 8.1 | |
| | | | |
| TOTAL | 125.4 | 124.1 | |
| | | | |
| | | | |
| % protein | | 81 | 14.31 |
| mgSA/gm protein | | 52 | 29.92 |
| mgSA/gm powder | 138.03 | 42.12 | 4.28 |
| (Repeat analysis) | 117.02 | | |
| Average | 127.525 | | |
| | | | |
| Amino acid levels are expressed as grams of the amino acid per 16 grams of nitrogen | | | |
| CGMP Powder is commercial glycomacropeptide from Tatua Co-Operative Dairy Company Ltd. | | | |
| ED Whey Powder is commercial electrodialyzed whey powder | | | |
| Samples W4731, W4733, and W4735 are samples of enhanced sialic acid CGMP with reduced threonine available from Tatua Co-Operative Dairy Company Limited, Tatuanui, Morrinsville, New Zealand. | | | |

It is noted that the threonine level of the novel product are about one-fourth that of commercial CGMP. Accordingly, it is believed that use of a high-sialic acid CGMP with reduced threonine in an infant formula can provide a formula having a high level of sialic acid at normal, desirable protein levels of about 14 g protein/ liter, and yet provide a desirable amino acid profile and low levels of threonine.

### EXAMPLE 6.

This illustrates the use of a CGMP fraction having enhanced levels of sialic acid in an infant formula.

The CGMP product having enhanced levels of sialic acid can be used as a protein source in an infant formula in the same manner as a whey powder or normal CGMP powder. By way of example, Table 11 shows the sialic acid content that could be expected for an infant formula in which the protein content is supplied by conventional sources. The amount of CGMP powder that is used is limited in order to avoid undue deviation of the amino acid profile of the protein that is provided from a desirable infant standard profile.

**Table 11: Sialic acid content of infant formula with protein provided by conventional sources:**

| Protein Source | mg sialic acid/ gm protein | Percent protein | grams protein/liter | mg sialic acid/liter |
|---|---|---|---|---|
| Whey protein concentrate | 23 | 35% | 6.82 | 156.77 |
| Nonfat dry milk, low heat | 6.37 | 34% | 6.25 | 39.8 |
| CGMP Powder | 52 | 81% | 1.11 | 57.62 |
| Total | | | 14.17 | 254.18 |

The CGMP powder that is used in this formulation can be replaced by novel CGMP product having an enhanced level of sialic acid, as is described above in Reference Example 3. Table 12 shows that when this is done, the sialic acid content of the formulation is more than doubled with no further disruption of the amino acid profile of the protein.

**Table 12: Sialic acid content of infant formula with protein provided by conventional sources plus a CGMP product having an enhanced level of sialic acid:**

| Protein Source | mg sialic acid/ gm protein | Percent protein | grams protein/liter | mg sialic acid/liter |
|---|---|---|---|---|
| Whey protein concentrate | 23 | 35% | 6.82 | 156.77 |
| Nonfat dry milk, low heat | 6.37 | 34% | 6.25 | 39.8 |
| CGMP Product with enhanced sialic acid | 214 | 54.93% | 1.11 | 237.54 |
| Total | | | 14.17 | 434.12 |

If the CGMP powder having enhanced sialic acid levels were to be used at double the levels described above at the expense of nonfat dry milk, the sialic acid content of the formula could be increased to the level shown in Table 13.

**Table 13: Sialic acid content of infant formula with protein provided by conventional sources plus a CGMP product with an enhanced level of sialic acid:**

| Protein Source | mg sialic acid/ gm protein | Percent protein | grams protein/liter | mg sialic acid/liter |
|---|---|---|---|---|
| Whey protein concentrate | 23 | 35% | 6.82 | 156.77 |
| Nonfat dry milk, low heat | 6.37 | 34% | 5.14 | 32.74 |
| CGMP Product with enhanced sialic acid | 214 | 54.93% | 2.22 | 475.08 |
| Total | | | 14.18 | 664.59 |

### EXAMPLE 7.

This example illustrates the efficacy of a diet containing casein glycomacropeptide on the learning behavior and memory behavior of piglets.

### Methods

### Animals

Over a period of 20 months, 3-day-old male domestic piglets (Sus scrofa) weighing 1.5 to 2.4 kg from 14 different litters were purchased from a commercial piggery. They were stratified according to weight and randomly allocated to 1 of 4 treatments. Piglets were housed in pairs according to treatment in wire pens with concrete flooring in a temperature-controlled room. The home pens contained a 'nest' (a rubber tire covered with a towel), a heat lamp and an identical plastic toy. The piglets were encouraged to use the far end of the pen as a toilet and the pens were cleaned daily. The lights were maintained on a 12 hr light (8 am - 8 pm)/ dark (8 pm - 8 am) cycle.

### CGMP supplementation

Casein glycomacropeptide (CGMP) containing 60 mg/g of sialic acid was supplied by Tatua Dairy Cooperative (Morrinsville, New Zealand) and blended into the pig's milk replacer at specified levels by Wombaroo Food Products (Glen Osmond, Australia). The amount of sialic acid in the final milk varied according to group: 77 mg/L (group 1, the control group with no added CGMP), 250 mg/L (group 2, low dose), 600 mg/L (group 3, middle dose) and 842 mg/L (group 4, highest dose). These levels represented an intake of 25, 71, 171 and 240 mg/kg body weight/day respectively. The replacers were formulated so that total protein intake remained the same irrespective of the amount of added CGMP. To maintain normal rates of growth, the piglets received 285 ml milk/kg in the first 2 weeks of study and 230 ml/kg in the remaining weeks. Feeding times were at 8:00, 13:00, 18:00 and 22.30, with an extra 50 ml milk supplied at night. Body weight, milk intake and medication were recorded daily.

### Learning ability and memory performance assessment

Formal learning tests began at 23 days of age using an 8-arm radial maze (FIG. 1 and FIG.2). The maze was a purpose-built wooden structure situated in a learning area (4.2 m x 4.4 m) adjacent to the home pens. A video camera was installed overhead to record the learning and memory tests. In order to reduce stress, piglet pairs were allowed to explore the maze one day before the start of formal testing. Two learning tests were performed: task 1 and task 2. Both tests had accessible milk in one arm, and inaccessible milk in the remaining 7 arms, such that all 8-arms of the maze gave the same olfactory signals. In both tests, a visual cue consisting of 3 black dots was placed randomly on a door with accessible milk in the arm (FIG. 3). In task 1 (the easy task), one black dot was placed on the remaining 7 doors. In task 2 (the more difficult one), two black dots were placed on the remaining 7 doors.

All piglets were tested in the maze individually. Forty trials of task 1 were conducted over 5 days (8 trials/day) and 40 trials of task 2 over 6 days. Assessment of learning speed was determined as the number of trials taken to successfully learn the visual cue and the number of mistakes (wrong door) and successes (correct door) in finding the accessible milk arm during each trial. A mistake was registered each time the piglet entered or put its whole head through the wrong door. A success was registered only when piglets entered the correct door and found the accessible milk. The criterion to learn the visual cue was a maximum of 1 mistake in 3 consecutive trials. Two days after completion of the task 1 trials and task 2 trials, the same task was presented as a 'memory test' for one trial only. All the tests were conducted by 2 trained staff who were not blinded to the level of sialic acid intake, but the results were later corroborated by independent analysis of the video material.

### Assessment of stress

Because stress may influence learning and memory, morning blood cortisol concentration was measured at weekly intervals beginning on day 7 using a commercial kit (Coat-A-Count Cortisol, Diagnostic Products, Doncaster, Australia). Inter-assay and intra-assay variation were 9% and 12% respectively.

### Analysis of ganglioside-bound and protein-bound sialic acid

On day 34 or 35, the piglets were euthanased by sodium pentothal injection (50 mg/kg). Brain tissues from the cerebral frontal cortex were collected and stored at -80° until analyzed. Ganglioside-bound and protein-bound sialic acids were determined separately using published methods (See, Mahal, *Id*.). All samples were analyzed in duplicate and the final concentration of sialic acid in each fraction expressed in µg/g wet tissue.

### Statistical analysis

Differences in learning speed (number of trials to learn the visual cue) were compared using Kaplan-Meier survival analysis with Cox regression to examine potential covariates that may influence learning speed. Comparisons between means (with or without covariates) were performed using the general linear model (Univariate ANOVA). Pearson's correlation was used to examine the relationship between number of mistakes, successes, body weight and memory performance. All statistical analyses were completed using SPSS for Windows 11 Inc, Chicago. A significance level of 0.05 was used.

### Results

### Learning speed

In both the easy and more difficult task, the sialic acid-supplemented groups learned the visual cue faster than the control group (Kaplan-Meier, P = 0.0014 in task 1 and P = 0.0177 in task 2, see FIG. 4 and FIG. 5). In task 1, only 45% of the control group reached criterion within 40 trials while the best learning performance was in group 2 (100% learned within 40 trials) followed by the group 4 (80%) and group 3 (70%). In task 2, there was a dose-response relationship (P = 0.0177), with all piglets in group 4 reaching criterion within 30 trials. The differences among groups were still significant when adjusted for differences in body weight at the time of the trial (P < 0.05).

### Covariates for learning

It is likely that the piglets used the information they obtained in task 1 to help them to learn the visual cue in task 2. We therefore used the total number of mistakes and successes in task 1 as covariates for the analysis of the learning speed in task 2. The difference between groups after adjustment remained highly significant (P = 0.002 mistakes as covariates & 0.004 successes as covariates) and the dose-response order in the learning speed of task 2 was not changed. The findings were similar if all 40 trials in task 1 were considered or just the last 20 trials. The number of mistakes in trials 21 - 40 of task 1 also varied significantly among the groups. This was the case with or without the use of mistakes in trials 1-10 as a covariate (P = 0.016). The control group made significantly more mistakes than group 2 (P = 0.005) and group 4 (P= 0.006), but only marginally more than group 3 (P = 0.06). In a similar analysis for task 2, the covariate was significant, but the groups were not significantly different (with or without the covariate) (P > 0.05) (FIG. 6). Similar analyses using the total number of mistakes in all trials revealed a significant difference between the treatment groups (combined groups 2, 3 & 4) vs the control group (P= 0.009) and a marginal difference between the groups in task 2 (p = 0.048).

### Memory test

The piglets that were able to reach the learning criterion were assessed for their ability to remember the visual cue 48 hr later. The overall difference between the groups was statistically significant for task 2 (P = 0.036), but not task 1 (P = 0.165, FIG. 7(A)).

There was no dose-response effect in either task. In task 2, group 3 and the control were approximately equivalent (FIG. 7(B)). Combining groups 2, 3 and 4 as the treated group, there were 35% fewer mistakes (mean = 1.5) compared with the control group (mean = 2.4, p = 0.036).

Not surprisingly, the number of mistakes and successes during the learning period significantly influenced memory performance in all piglets. Thus a higher number of mistakes in the learning period predicted a higher number of mistakes in the memory test (P = 0.03 in task 1 and P= 0.029 in task 2, Spearman's correlations 2-tailed). Similarly, a higher number of successes in the learning period was significantly associated with a lower number of mistakes in the memory test in task 1(P = 0.007), but not task 2 (P = 0.20). Body weight, rate of weight gain and learning speed did not significantly affect performance in the memory test (P> 0.05).

### Plasma cortisol

The mean cortisol concentration in each group over the five-week study period is shown in FIG. 8(A). Across all groups, the difference between the first week and all other weeks was significantly different (week 1 vs weeks 2, 3, 4 and 5; P = 0.031, 0.015, 0.001, and 0.001 respectively). From week 2 onwards, the cortisol concentrations for all piglets decreased on average, yet not significantly (P > 0.05). When all supplemented groups were combined into one group, the difference between treatment vs control group was not significant (P > 0.05, FIG. 8(B)). In week 2, the difference between group 2 and group 3 was significant (P = 0.044). When blood plasma cortisol concentration was used as a covariate during learning, no significant effects were observed (P > 0.05).

### Brain sialic acid concentration

The majority of sialic acid in piglet brain frontal cortex (86%) was bound to gangliosides with a small fraction (∼13%) bound to glycoprotein and less than 2% in the free form. This is comparable to previous published work by Brunngraber, E. G. et al., Brain Res., 38:151-162 (1972), and Mahal, L. K. et al., in J. Biolo. Chem., 277:9255-9261 (2002). There was a significant dose-response relationship between brain protein-bound sialic acid and CGMP supplementation levels, e.g., group 4 contained the highest level, followed by group 3, 2 and 1. The level in group 1 was significantly lower than group 4 (P = 0.001) and group 3 (P = 0.001) but not group 2 (P = 0.126). On average, protein-bound sialic acid in grey matter was 6-10% higher in CGMP supplemented groups vs the control (P = 0.000, Table 14).

**Table 14: The mean level of ganglioside-bound, protein-bound and free sialic acid in brain frontal cortex according to level of level of CGMP supplementation.**

| Group | n^{a} | Ganglioside-bound (µg/g) | | Protein-bound (µg/g) | | Free (µg/g) | | Total (µg/g) | |
|---|---|---|---|---|---|---|---|---|---|
| | | Mean | SE | Mean | SE | Mean | SD | Mean | SE |
| Group 1 | 14 | 162 | 9 | 114 | 2 | 3.0 | 0.2 | 279 | 10 |
| Group 2 | 13 | 176 | 9 | 121 | 2 | 3.1 | 0.2 | 300 | 10 |
| Group 3 | 14 | 182 | 9 | 126 | 2 | 3.0 | 0.2 | 311 | 10 |
| Group 4 | 12 | 185 | 10 | 127 | 2 | 3.2 | 0.2 | 315 | 10 |
| P value | | 0.307 | | 0.001 | | 0.876 | | 0.068 | |

| | | | | | | | | | |
|---|---|---|---|---|---|---|---|---|---|
| Notes: a. "n" is the number of piglets per group. | | | | | | | | | |

The average concentration of ganglioside-bound sialic acid also followed a dose-response order (13%, 11% and 8% higher in group 4, group 3 and group 2 respectively vs the control), but the differences did not reach statistical significance (P = 0.089, 0.124 and 0.299 respectively, Table 14). When all CGMP supplemented groups were combined into one group, the difference between treatment vs control group was marginally significant (P = 0.07, FIG. 9). Inter-individual variation in ganglioside-bound sialic acid was larger than that of protein-bound sialic acid (CV = ∼5% in ganglioside-bound vs 2% in protein-bound sialic acid). The same phenomenon has been reported in a study of human infant brains reported by Mahal, L. K., *Id..*

Total sialic acid concentration (ganglioside-bound + protein-bound) was marginally higher in the combined CGMP treatment groups vs control group (P = 0.051), but free sialic acid was not (P = 0.58, FIG. 9).

### Correlation of brain sialic acid with learning performance

A higher concentration of protein-bound, ganglioside-bound and total sialic acid in brain frontal cortex was associated with faster learning in both task 1 and task 2 (see FIG. 10(A), FIG. 10(B), FIG. 11 (A), and FIG. 11 (B)).

None of the correlations, whether parametric or non-parametric, reached statistical significance (see all FIGS. 10 and FIGS. 11). In a similar analysis for the memory task, a negative correlation (high sialic acid content, fewer mistakes) was found in both task 1 and task 2, but again the results did not reach statistical significance (P > 0.05), except for the protein-bound form in group 4 for task 1 (P= 0.045, FIG. 12). There was a significant positive correlation between total number of successes and ganglioside-bound sialic acid in task 1 (P = 0.045), but not in task 2 (P < 0.05).

### Body weight gain

Mean (± SE) starting body weight was the same in each group (2.1 ± 0.04 kg) and the animals gained weight at similar rates (FIG. 13). Although the control group weighed slightly more than the other groups at the end of the study, the rate of weight gain (g/day) did not vary significantly among the groups (P = 0.503). The discussion of the references herein is intended merely to summarize the assertions made by their authors and no admission is made that any reference constitutes prior art. Applicants reserve the right to challenge the accuracy and pertinency of the cited references.

In view of the above, it will be seen that the several advantages of the invention are achieved and other advantageous results obtained.

## Claims

1. A non-therapeutic method for improving learning and/or memory behavior in a human infant comprising administering to the infant casein glycomacropeptide in an amount sufficient to improve learning and/or memory behavior in the infant, wherein the amount sufficient to improve learning and/or memory behavior in the infant provides at least 100 mg/kg/day of sialic acid to the infant.

2. The method according to claim 1, wherein said infant is administered an infant formula comprising said casein glycomacropeptide.

3. The method according to claim 1, wherein the infant is between about 1 day and about 4 years of age.

4. The method according to claim 1, wherein the infant is a neonatal infant.

5. The method according to claim 1, wherein the need of improvement in learning and/or memory behavior is shown where substantially all of the infant's nutritional requirement has been supplied by administration of a formula having less than about 100 mg/L of sialic acid.

6. The method according to claim 5, wherein a major part of the protein contained in said formula having less than 100 mg/L of sialic acid is soy protein or cow's milk protein.

7. The method according to claim 6, wherein at least about 75% by weight of the protein contained in said formula having less than 100 mg/L of sialic acid is soy protein or cow's milk protein.

8. The method according to claim 6, wherein substantially all of the protein contained in said formula having less than 100 mg/L of sialic acid is soy protein or cow's milk protein.

9. The method according to claim 2, wherein the infant formula is a nutritionally complete infant formula comprising carbohydrate, lipid, and protein.

10. The method according to claim 9, wherein the protein comprises a material that is selected from cow's milk protein, soy protein, and mixtures thereof.

11. The method according to claim 1, wherein the amount of casein glycomacropeptide is sufficient to provide at least 200 mg/kg/day of sialic acid to the infant.

12. The method according to claim 2, wherein the infant formula comprises a total protein content of between 12 and 16 grams/liter of which no more than 40% by weight is provided by casein glycomacropeptide.

## Patentansprüche

1. Nichttherapeutisches Verfahren zur Verbesserung von Lern- und/ oder Gedächtnisverhalten eines menschlichen Kleinkinds, umfassend die Verabreichung von Kaseinglykomakropeptid in einer ausreichenden Menge an das Kleinkind, um das Lern- und/ oder Gedächtnisverhalten des Kleinkinds zu verbessern, wobei die ausreichende Menge zur Verbesserung des Lern- und/ oder Gedächtnisverhaltens des Kleinkinds das Kleinkind mit mindestens 100 mg Sialinsäure/kg/Tag versorgt.

2. Verfahren nach Anspruch 1, wobei dem Kleinkind eine Säuglingsnahrung verabreicht wird, umfassend das Kaseinglykomakropeptid.

3. Verfahren nach Anspruch 1, wobei das Kleinkind zwischen ungefähr 1 Tag und ungefähr 4 Jahren alt ist.

4. Verfahren nach Anspruch 1, wobei das Kleinkind ein frühgeborenes Kleinkind ist.

5. Verfahren nach Anspruch 1, wobei das Bedürfnis zur Verbesserung des Lern- und/ oder Gedächtnisverhaltens gezeigt wird, wo im Wesentlichen der gesamte Nährstoffbedarf des Kleinkindes durch Verabreichung einer Säuglingsnahrung mit weniger als etwa 100 mg/L Sialinsäure zugeführt worden ist.

6. Verfahren nach Anspruch 5, wobei ein wesentlicher Bestandteil des in der Säuglingsnahrung mit weniger als 100 mg/L Sialinsäure enthaltenen Proteins Sojaprotein oder Kuhmilchprotein ist.

7. Verfahren nach Anspruch 6, wobei mindestens ungefähr 75 Gew.-% des in der Säuglingsnahrung mit weniger als 100 mg/L Sialinsäure enthaltenen Proteins Sojaprotein oder Kuhmilchprotein ist.

8. Verfahren nach Anspruch 6, wobei im Wesentlichen das gesamte in der Säuglingsnahrung mit weniger als 100 mg/L Sialinsäure enthaltene Protein Sojaprotein oder Kuhmilchprotein ist.

9. Verfahren nach Anspruch 2, wobei die Säuglingsnahrung eine diätisch vollständige Säuglingsvollnahrung ist, umfassend Kohlehydrat, Fett und Protein.

10. Verfahren nach Anspruch 9, wobei das Protein einen Stoff umfasst, ausgewählt aus Kuhmilchprotein, Sojaprotein und Gemischen davon.

11. Verfahren nach Anspruch 1, wobei die Menge an Kaseinglykomakropeptid ausreichend ist, um das Kleinkind mit mindestens 200 mg Sialinsäure/kg/Tag zu versorgen.

12. Verfahren nach Anspruch 2, wobei die Säuglingsnahrung einen Gesamtproteinanteil von zwischen 12 bis 16 Gramm/Liter aufweist, wovon nicht mehr als 40 Gew.-% durch Kaseinglykomakropeptid bereitgestellt wird.

## Revendications

1. Méthode non-thérapeutique d'amélioration de l'apprentissage et/ou du comportement de la mémoire chez un nourrisson humain, comprenant l'administration au nourrisson de glycomacropeptide de caséine en une quantité suffisante pour améliorer l'apprentissage et/ou le comportement de la mémoire chez le nourrisson, dans laquelle la quantité suffisante pour améliorer l'apprentissage et/ou le comportement de la mémoire chez le nourrisson prévoit au moins 100 mg/kg/jour d'acide sialique au nourrisson.

2. Méthode selon la revendication 1, dans laquelle une formule infantile comprenant du glycomacropeptide de caséine est administrée au nourrisson.

3. Méthode selon les revendications 1, dans laquelle le nourrisson a un âge compris entre environ un jour et environ quatre ans.

4. Méthode selon les revendications 1, dans laquelle le nourrisson est un nouveau-né.

5. Méthode selon la revendication 1, dans laquelle le besoin d'amélioration du comportement de l'apprentissage et/ou de la mémoire est indiqué, où sensiblement la totalité des besoins nutritionnels du nourrisson ont été satisfaits par l'administration d'une formule ayant moins 100 mg/L environ d'acide sialique.

6. Méthode selon la revendication 5, dans laquelle une majeure partie de la protéine contenue dans ladite formule ayant moins de 100 mg/L d'acide sialique est de la protéine de soja ou de la protéine du lait de vache.

7. Méthode selon la revendication 6, dans laquelle au moins environ 75 % en poids de la protéine contenue dans ladite formule ayant moins de 100 mg/L d'acide sialique est de la protéine de soja ou de la protéine du lait de vache.

8. Méthode selon la revendication 6, dans laquelle sensiblement la totalité de la protéine contenue dans ladite formule ayant moins de 100 mg/L d'acide sialique est de la protéine de soja ou de la protéine du lait de vache.

9. Méthode selon la revendication 2, dans laquelle la formule infantile est une formule infantile nutritionnellement complète comprenant des glucides, des lipides et des protéines.

10. Méthode selon la revendication 9, dans laquelle la protéine comprend un matériau qui est sélectionné parmi de la protéine de lait de vache, de la protéine de soja, et des mélanges de ceux-ci.

11. Méthode selon la revendication 1, dans laquelle la quantité de glycomacropeptide de caséine est suffisante pour fournir au moins 200 mg/kg/jour d'acide sialique au nourrisson.

12. Méthode selon la revendication 2, dans laquelle la formule infantile comprend une teneur totale en protéines comprise entre 12 et 16 grammes/litre, dont pas plus de 40 % en poids sont fournis par le glycomacropeptide de caséine.
